# EUROPÄISCHE PATENTSCHRIFT

(11) **EP 3 333 143 B1**
(45) Veröffentlichungstag und Bekanntmachung des Hinweises auf die Patenterteilung: **07.08.2019**
(21) Anmeldenummer: 17204866.2
(22) Anmeldetag: 01.12.2017
(51) Int. Cl.: C07C 5/333, C07C 11/02

(54) **DEHYDRIERUNG OLEFINREICHER KOHLENWASSERSTOFFGEMISCHE**
DEHYDROGENATION OF OLEFIN-RICH HYDROCARBON MIXTURES
DÉSHYDROGENATION DE MÉLANGES D'HYDROCARBURES RICHES EN OLÉFINES

(30) Priorität: 08.12.2016 EP 16202840
(43) Veröffentlichungstag der Anmeldung: 13.06.2018
(73) Patentinhaber: Evonik Degussa GmbH, 45128 Essen (DE)
(72) Erfinder: Stochniol, Guido, 45721 Haltern am See (DE); Franke, Robert, 45772 Marl (DE)

(56) Entgegenhaltungen:
- DE-A1- 10 229 661
- NEIL M. SCHWEITZER ET AL: "Propylene Hydrogenation and Propane Dehydrogenation by a Single-Site Zn 2+ on Silica Catalyst", ACS CATALYSIS, Bd. 4, Nr. 4, 4. April 2014 (2014-04-04), Seiten 1091-1098, XP055370107, US ISSN: 2155-5435, DOI: 10.1021/cs401116p

## Beschreibung

Die Erfindung befasst sich mit der Frage, wie Gemische von Alkanen mit zwei bis fünf Kohlenstoffatomen dehydriert werden können, wenn das Gemisch einen hohen Anteil an Olefinen aufweist.

Kohlenwasserstoffe sind chemische Verbindungen, die ausschließlich aus Kohlenstoff und Wasserstoff bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet. Aufgrund der unterschiedlichen Bindungstypen sind die Alkene deutlich reaktiver als die Alkane. Deswegen sind Alkene chemisch besser nutzbar und entsprechend wertvoller als Alkane.

In der organischen Chemie werden Kohlenwasserstoffe häufig nach der Anzahl ihrer Kohlenstoffatome pro Molekül bezeichnet, indem der jeweiligen Substanzklasse das Präfix Cₙ vorangestellt wird. Dabei bezeichnet n die jeweilige Anzahl der Kohlenstoffatome in einem Molekül. So sind C₄-Olefine zu verstehen als Substanzen der Klasse der Alkene mit vier Kohlenstoffatomen. C₈-Olefine weisen dementsprechend acht Kohlenstoffatome pro Molekül auf. Soweit im Folgenden das Präfix Cₙ₊ verwendet wird, ist von einer Substanzklasse die Rede, die mehr als n Kohlenstoffatome pro Molekül aufweist. Ein C₄₊-Olefin hat demnach mindestens fünf Kohlenstoffatome.

Aufgrund unterschiedlicher Anordnungs- und Verknüpfungsmöglichkeiten der Kohlenstoff- und Wasserstoffatome existieren innerhalb den hier besprochenen Substanzklassen mehrere Isomere, welche dieselbe Anzahl an Kohlenstoffatomen aufweisen. So existieren beispielsweise zwei Alkane mit jeweils vier Kohlenstoffatomen, nämlich n-Butan und Isobutan. Da bei den Alkenen die Kombinationsvielfalt größer ist, sind noch mehr Isomere möglich. So kommen insgesamt vier Olefine mit vier Kohlenstoffatomen vor, nämlich Isobuten, 1-Buten, cis-2-Buten und trans-2-Buten. Die drei linearen Butene 1-Buten, cis-2-Buten und trans-2-Buten werden oft zusammenfassend als n-Buten bezeichnet. Bei den C₃-Kohlenwasserstoffen gibt es hingegen jeweils nur ein Isomer, nämlich das Alkan mit drei Kohlenstoffatomen Propan und das C₃-Alken Propen. Bei den längerkettigen Kohlenwasserstoffen C₅₊ nimmt die Vielfalt der Isomere stark zu. Trotz der identischen Anzahl an Kohlenstoffatomen weisen Isomere unterschiedliche Stoffeigenschaften auf, die für ihre industrielle Nutzung relevant sind.

Die C₄-Chemie befasst sich mit der Herstellung von Spezialchemikalien aus Butenen. Eine Einführung bieten:
Geilen, F. M., Stochniol, G., Peitz, S. and Schulte-Koerne, E.: Butenes. Ullmann's Encyclopedia of Industrial Chemistry. 1-13. Published Online: 31 JAN 2014 DOI: 10.1002/14356007.a04_483.pub3

Als Rohstoffquelle werden heute meist so genannte C₄-Schnitte eingesetzt, die als "Crack-C4" aus Steamcrackern oder als "FCC-C4" aus fluid-katalytischen Crackern stammen. Solche Cracker werden im Wesentlichen mit Naphtha bzw. VGO (Vacuum Gas Oil) beschickt, welche wiederum aus der Destillation von Rohöl stammen. Da Crack-C4 und FCC-C4 in der Wertschöpfungskette der Petrochemie Produkte von Crackprozessen sind, sind die Preise für diese Rohstoffe aufgrund ihrer Abhängigkeit vom Erdölpreis entsprechend volatil. Darüber hinaus geht die Verfügbarkeit von hochwertigen Crack-C4 ständig zurück, da die Fahrweise der Streamcracker zu Ungunsten der C₄-Ausbeute auf die Produktion der C₂- und C₃-Olefine Ethen und Propen hin optimiert wird.

Deswegen besteht in der C₄-Chemie ein grundsätzliches Interesse daran, anstelle der klassischen Rohstoffquellen auch alternative Rohstoffe nutzbar zu machen.

Eine Möglichkeit bietet hier die Technologie der Dehydrierung. Die Dehydrierung ist eine chemische Reaktion, bei der aus einem Kohlenwasserstoff Wasserstoff entfernt wird. Konkret können so aus Alkanen unter Freisetzung von Wasserstoff (H₂) Alkene hergestellt werden. Die Anzahl der Kohlenstoffatome der erzeugten Alkene entspricht dann denen der eingesetzten Alkane. Da die Alkane reaktionsträger sind als die Alkene, muss für die Dehydrierung Energie aufgewandt werden. Diese ist der Reaktion in Form von Wärme zuzuführen. Im Interesse einer Energieersparnis findet die industrielle Dehydrierung stets in Gegenwart von festen Katalysatoren statt.

Die Technologie zur Dehydrierung von Alkanen unterscheidet oxidative Verfahren und nicht oxidative Verfahren. Bei der oxidativen Dehydrierung wird dem Alkangemisch ein Oxidationsmittel wie Sauerstoff oder Luft zugegeben, um den Wärmebedarf der stark endothermen Dehydrierung zumindest teilweise aus der Oxidation des frei gesetzten Wasserstoffs zu gewährleisten. Bei der nicht oxidativen Dehydrierung wird indes auf die Zugabe von Oxidationsmitteln verzichtet und stattdessen die benötigte Wärme von extern in den Reaktor eingebracht, etwa durch Beheizen mit einem Brenngas (meist Methan, Erdgas, Spaltgase aus dem Dehydrierprozess und ggf. teilweise Beimischung von Wasserstoff, der in der Dehydrierung gebildet wird). Beide Prozessvarianten unterscheiden sich stark in der Zusammensetzung des Dehydrierungsgemisches. Eine detaillierte Abhandlung über die gängige Dehydrierungstechnologie findet sich in US2006/0122436A1.

Problematisch bei der industriell praktizierten Dehydrierung ist die Koksbildung auf dem Katalysator. Gemeint ist damit ein Niederschlag von Kohlenstoff auf der Oberfläche des Katalysators. Diese führt zu einer Desaktivierung des Katalysators, sodass dieser ausgetauscht oder regeneriert werden muss. Die Betriebskosten steigen dadurch stark an, sodass die Dehydrierung unwirtschaftlich wird.

Aus diesem Grund sind Alkene im Eintrittsbereich des Dehydrierkatalysators ungern gesehen, da sie wegen ihrer verglichen mit Alkanen höheren Reaktivität zu einer schnellen Koksablagerung auf dem Katalysator führen. Folglich raten die Anbieter kommerzieller Dehydrier-Prozesse davon ab, Alkene in die Dehydrierung zu fahren.

Sofern in dem zu dehydrierenden Einsatzstoffgemisch größere Mengen an Olefinen enthalten sind, müssen entsprechende Maßnahmen getroffen werden, um der Verkokung zu begegnen:
So beschreibt US5389342 den apparativen Aufbau eines Reaktors zur Dehydrierung von n-Butan und Isobutan. Ein flüssiges Einsatzstoffgemisch mit den zu dehydrierenden Alkanen wird in einem Verdampfer in die Gasphase überführt und sodann mit Wasserdampf verdünnt, um bei dem nun erfolgenden Kontakt mit dem Dehydrierkatalysator Koksablagerungen zu reduzieren und den Umsatz zu steigern.

Die Wasserdampfzugabe nutzt den Effekt der Kohlevergasung, nach der Kohlenstoff in Gegenwart von Wasserdampf in Synthesegas umgesetzt wird:

C+ H₂O -> CO + H₂

Nachteil ist, dass das reaktionsfreudige Synthesegas viele unerwünschte Nebenprodukte bildet, die erst wieder aufwendig aus dem Produktgemisch der Dehydrierung abgeschieden werden müssen.

Bei dem in US4926005 beschriebenen Verfahren wird ein C₂ bis C₅-Paraffingemisch vor einer nicht oxidativen Dehydrierung mit einem gebrauchten Dehydrier-Katalysator unter nicht dehydrierenden Bedingungen kontaktiert, um den Alkan-Umsatz zu steigen. Der Vorkontakt erfolgt bei Temperaturen zwischen 0 °C und 120 °C und in Abwesenheit von Wasserstoff und Sauerstoff. Möglicherweise werden dort S- oder N-Komponenten absorbiert. Von Olefinen im Eingang ist nicht die Rede.

US4013733 beschreibt, dass ein C₄ bis C₃₀-Paraffingemisch vor dem Kontakt mit dem Dehydrier-Katalysator mit Wasserstoff versetzt wird. Bei dem Kontakt herrschen Temperaturen zwischen 371 °C und 677 °C. Das Verhältnis von Wasserstoff zu Kohlenwasserstoff liegt zwischen 1 : 1 bis 20 : 1. Der Wasserstoff wird direkt in die Dehydrier-Zone eingespritzt. Zweck der Wasserstoffzugabe ist es, solche Zielsubstanzen herzustellen, welche dieselbe Anzahl an Kohlenstoffatomen wie die Ausgangsstoffe haben, jedoch eine reduzierte Anzahl an Wasserstoffatomen aufweisen. Olefine sind im Einsatzgemisch nicht enthalten.

Die Gruppe um Neil M Schweitzer erörtert ebenfalls das Problem Katalysator-Desaktvierung hervorgerufen von Kohlenstoff-Ablagerungen. Sie beschreibt ein Katalysatorsystem basierend auf Zink und Silica, welches sich sowohl für die Hydrierung von Propen als auch für die Dehydrierung von Propan eignet:
Schweitzer et al.: Propylene Hydrogenation and Propane Dehydrogenation by a Single-Site Zn2+ on Silica Catalyst. ACS Catal., 2014, 4 (4), pp 1091-1098. DOI: 10.1021/cs401116p

Die Hydrierung erfolgte bei 200 °C, die Dehydrierung bei 550 °C bzw. bei 650 °C. Ein Nachteil ist der hohe Wasserstoffüberschuss (molares Verhältnis Wasserstoff: Propen ist ca. 10 : 1) mit dem hier gearbeitet wird, da ein derartig hoher Überschuss die Gleichgewichtsreaktion Hydrierung ↔ Dehydrierung negativ beeinflussen kann, wobei insbesondere die Dehydrierung sehr ungünstig verlaufen würde. Deshalb werden die Hydrierung und Dehydrierung nicht wie hier aufeinanderfolgend durchgeführt, sondern unabhängig voneinander untersucht. Nachteilig ist weiterhin dass es sich bei Zink nicht um eine effektive Hydrierkomponente zur Hydrierung von Olefinen handelt, die für den Einsatz im industriellen Maßstab uneingeschränkt geeignet ist.

Die zum Anmeldezeitpunkt noch unveröffentlichte europäische Patentanmeldung 16188267.5 befasst sich mit der Dehydrierung von Flüssiggas (LPG). Vor der Dehydrierung ist optional eine Hydrierung vorgesehen, um den Olefingehalt des LPG auf einen Wert unter 1 Gew.-% abzusenken. Die Hydrierung erfolgt in der Flüssigphase.

Im Hinblick auf diesen Stand der Technik liegt der Erfindung die Aufgabe zu Grunde, ein Verfahren zur Dehydrierung von Alkanen anzugeben, bei der solche Einsatzgemische verwendet werden können, die einen hohen Anteil an Olefinen aufweisen, d. h. etwa 1 Gew.-% bis 10 Gew.-%. Konkret sollen Alkene mit zwei bis fünf Kohlenstoffatomen aus Alkanen mit derselben Kettenlänge erzeugt werden, die Anzahl der Kohlenstoffatome soll bei der Dehydrierung also nicht verändert werden. Das Verfahren soll im industriellen Maßstab realisierbar sein.

Gelöst wird die Aufgabe durch ein Verfahren mit den folgenden Schritten:
a) Bereitstellen eines flüssigen Einsatzstoffgemisches bei einem Druck zwischen 0.1^{∗}10⁵ Pa und 6.0^{∗}10⁵ Pa, wobei das Einsatzstoffgemisch Alkane mit zwei bis fünf Kohlenstoffatomen sowie Alkene mit zwei bis fünf Kohlenstoffatomen umfasst, und wobei der Massenteil der Alkene in dem Einsatzstoffgemisch bezogen auf dessen Gesamtmasse 1 Gew.-% bis 10 Gew.-% beträgt;
b) Verdampfen des Einsatzstoffgemisches durch Temperaturerhöhung;
c) Zugabe von Wasserstoff in das verdampfte Einsatzstoffgemisch dergestalt, dass das molare Verhältnis von Wasserstoff zu den im Einsatzstoffgemisch enthaltenen Alkenen zwischen 0.8 : 1 und 1.2 : 1 beträgt;
d1) Kontaktierten des verdampften, wasserstoffhaltigen Einsatzstoffgemisches mit einem festen Katalysator bei einer Temperatur zwischen 450 °C und 760 °C und einem Druck von 0.1^{∗}10⁵ Pa bis 6.0^{∗}10⁵ Pa unter Erhalt eines Produktgemisches, wobei der Massenteil der Alkene mit zwei bis fünf Kohlenstoffatomen in dem Produktgemisch bezogen auf dessen Gesamtmasse 30 Gew.-% bis 70 Gew.-% beträgt; oder
d2) Kontaktieren des verdampften, wasserstoffhaltigen Einsatzstoffgemisches mit einem ersten festen Katalysator und einem Druck zwischen 0.1^{∗}10⁵ Pa und 6^{∗}10⁵ Pa unter Erhalt eines Intermediats, wobei der Massenteil der Alkene in dem Intermediat bezogen auf dessen Gesamtmasse 0 Gew.-% bis 1 Gew.-% beträgt und wobei die Temperatur des verdampften, wasserstoffhaltigen Einsatzstoffgemisches und/oder des Intermediats erhöht wird; und
e) Kontaktierten des Intermediats mit einem zweiten festen Katalysator bei einer Temperatur zwischen 450 °C und 760°C und einem Druck von 0.1^{∗}10⁵ Pa bis 6.0^{∗}10⁵ Pa unter Erhalt eines Produktgemisches, wobei der Massenteil der Alkene mit zwei bis fünf Kohlenstoffatomen in dem Produktgemisch bezogen auf dessen Gesamtmasse 30 Gew.-% bis 70 Gew.-% beträgt.

Ein solches Verfahren ist ein Gegenstand der Erfindung.

Eine Grundidee der Erfindung besteht darin, die im Einsatzgemisch enthaltenden Alkene zu den entsprechenden Alkanen zu hydrieren, bevor sie mit dem Dehydrierkatalysator in Kontakt kommen. So wird eine unerwünschte Koksablagerung vermieden. Die Hydrierung erfolgt durch geringfügige Zugabe von Wasserstoff (80 % bis 120 % der stöchiometrisch erforderlichen Menge). Die Hydrierung erfolgt entweder an einem speziell dafür vorgesehenen Hydrierkatalysator, der sich von dem Dehydrierkatalysator unterscheidet, oder an dem Dehydrierkatalysator selbst.

Ein wichtiger Aspekt der Erfindung besteht darin, dass die Hydrierung (an dem Kontakt mit dem ersten Katalysator) in der Gasphase erfolgt. Zu diesem Zweck wird das flüssige Einsatzstoffgemisch zunächst verdampft und dann vor der Hydrierung der Wasserstoff eindosiert.

Die Zugabe von Wasserstoff in das gasförmige (verdampfte) Einsatzstoffgemisch hat den Vorteil, dass Löslichkeitsgrenzen des Wasserstoffes unbeachtlich sind: Aufgrund des mitunter hohen Anteils an Olefin, welches sich im Einsatzstoffgemisch befindet, ist auch eine große Menge Wasserstoff für eine vollständige Hydrierung erforderlich. Wollte man die Hydrierung in der flüssigen Phase durchführen, müsste der Wasserstoff in dem flüssigen Einsatzstoffgemisch gelöst werden, wobei entsprechende Löslichkeitsgrenzen beachtlich wären. Ein stark olefinisches Einsatzstoffgemisch könnte in der flüssigen Phase ggf. gar nicht vollständig hydriert werden, da sich die für die Hydrierung notwendige Menge Wasserstoff nicht in die flüssige Phase lösen ließe. Folglich würde es in der nachgeschalteten Dehydrierung zu Koksablagerungen durch nicht hydriertes Olefin kommen.

Ein weiterer wichtiger Aspekt der Erfindung ist, dass die Hydrierung auf demselben Druckniveau erfolgt wie die Dehydrierung, also zwischen 0.1^{∗}10⁵ Pa und 6.0^{∗}10⁵ Pa. Bevorzugt wird der gesamte Verfahren isobarisch durchgeführt, also das Einsatzstoffgemisch bereits bei Reaktionsdruck (der Dehydrierung) bereitgestellt und dann dieser Druck auch während der Verdampfung und der Hydrierung aufrechterhalten.

Grund dafür ist, dass die Hydrierung bei diesen hohen Drücken nämlich die Gleichgewichtsreaktion zu der Dehydrierung darstellt: Bei geringen Temperaturen wird die Hydrierung begünstigt, bei hohen Temperaturen die Dehydrierung. Folglich wird bei dem erfindungsgemäßen Verfahren die Temperatur im Zuge der Hydrierung angehoben, sodass erst nach der Hydrierung die hohe Dehydriertemperatur vorliegt, welche die Dehydrierung an dem zweiten Katalysator begünstigt.

Die Nutzung des Dehydrierkatalystors zum Hydrieren der Olefine beruht demnach auf der Erkenntnis, dass die Hydrierung und Dehydrierung Gleichgewichtsreaktionen sind, die sich thermodynamisch in eine gewünschte Richtung beeinflussen lassen. Konkret begünstigen milde Temperaturen (20 °C bis 220 °C) die Hydrierung, währenddessen bei höheren Temperaturen (450 °C bis 760 °C) die Dehydrierung dominiert. Dementsprechend werden erfindungsgemäß die thermodynamischen Bedingungen bei dem ersten Kontakt (zur Hydrierung) und bei dem zweiten Kontakt (zur Dehydrierung) so angepasst, dass das Gleichgewicht in die gewünschte Richtung verschoben wird.

Genauer gesagt, besteht das Anpassen der thermodynamischen Bedingungen in der Erhöhung der Temperatur. Dies geschieht dadurch, dass entweder das Intermediat (also das hydrierte Einsatzgemisch) erwärmt wird und/oder darin, dass das Einsatzgemisch bereits bei seinem Kontakt mit dem ersten Katalysator erwärmt wird. Dabei ist zu beachten, dass die Hydrierung exotherm ist und insoweit auch die freiwerdende Reaktionswärme der Hydrierung genutzt werden kann, um das entstehende Intermediat vorzuheizen. Mittel zum Beheizen des Intermediats bzw. des wasserstoffhaltigen Einsatzstoffgemisches sind deswegen nicht zwingend erforderlich. Die Dehydriertemperatur (zwischen 450 °C und 760 °C) darf in Gegenwart des ersten Katalysators allerdings nicht erreicht werden, da der erste Katalysator dann dehydrierend wirken und rasch mit Koks belegt werden würde.

In einer grundlegen Variante der Erfindung sind der erste Katalysator und der zweite Katalysator identisch. Dies bedeutet, dass in der Hydrierung und in der Dehydrierung derselbe feste Katalysator eingesetzt wird. Dies senkt die Katalysatorkosten des Verfahrens, da nur eine katalytisch aktive Substanz für beide Prozessschritte gehandhabt werden muss. Dies setzt voraus, dass der Katalysator sowohl die Hydrierung (und bei erhöhter Temperatur) die Dehydrierung katalysiert.

Für diesen Zweck geeignet sind grundsätzlich solche Trägerkatalysatoren, die ein Trägermaterial und darauf aufgebracht eine hydrieraktive Komponente aufweisen. Als hydrieraktive Komponente kommen solche Elemente in Betracht, die in den Gruppen 8, 9 und 10 des Periodensystems der Elemente gemäß IUPAC Konvention aufgeführt sind. Besonders geeignet sind die Elemente Zinn und Zink. Besonders bevorzugt weisen der (erste und zweite) feste Katalysator ein Trägermaterial sowie zumindest Zinn und/oder Zink auf. Neben oder statt Zinn und/oder Zink können auch weitere hydrieraktive Komponenten enthalten sein, wie etwa Nickel, Platin, oder Palladium.

Als Trägermaterial eignet sich wahlweise Siliciumdioxid oder Aluminiumoxid. Es kann auch eine chemische oder physikalische Mischung aus Siliciumdioxid und Aluminiumoxid als Trägermaterial eingesetzt werden. Chemische Mischungen aus Siliciumdioxid und Aluminiumoxid werden oft als Silica/Alumina bezeichnet. Es können sowohl amorphe Silica/Alumina als Trägermaterial eingesetzt werden als auch kristalline (so genannte Zeolithe). Als Trägermaterial eignen sich auch Aluminate, welches gebildet ist aus Aluminiumoxid und einem Erdalkalimetall wie beispielsweise Calcium. Als Trägermaterial für einen kombinierten Hydrier/Dehydrierkatalysator ist im Übrigen auch Hydrotalcit geeignet.

Ein als erster und zweiter Katalysator besonders geeignetes Zinn/Zink-System auf Calciummodifiziertem Aluminiumoxid bzw. dessen Herstellung und Einsatz in der Dehydrierung ist in US4152365, US4926005 und US5151401 offenbart. Dieser Katalysator enthält zusätzlich auch Platin.

Eine zweite grundlegende Variante der Erfindung sieht vor, dass für die Hydrierung und für die Dehydrierung unterschiedliche Katalysatoren eingesetzt werden. Der erste und der zweite feste Katalysator sind demnach nicht identisch. Vorteil davon ist, dass die Katalysatoren für ihre jeweilige Aufgabe optimiert werden können.

Als erster fester Katalysator wird vorzugsweise ein Trägerkatalysator verwendet, der ein Trägermaterial und darauf aufgebracht mindestens ein Element ausgewählt aus der Gruppe bestehend aus Nickel, Platin und Palladium umfasst, also ein Katalysator der die Hydrierung besonders vorteilhaft beeinflussen kann.

Als Trägermaterial eignet sich wiederum Siliciumdioxid oder Aluminiumoxid oder eine physikalische oder chemische Mischungen daraus; ebenso wie oben zu dem kombinierten Katalysator ausgeführt. Als zweiter Katalysator eignet sich dann solch ein System, wie oben als erster und zweiter Katalysator beschrieben.

Das Verfahren ist dafür bestimmt, Einsatzstoffgemische zu verarbeiten, welche die folgende Spezifikation aufweisen:

| | |
|---|---|
| • Propan: | 0 Gew.-% bis 50 Gew.-%; |
| • Isobutan: | 0 Gew.-% bis 100 Gew.-%; |
| • n-Butan: | 0 Gew.-% bis 100 Gew.-%; |
| • Propen: | 0 Gew.-% bis 10 Gew.-%; |
| • Isobuten: | 0 Gew.-% bis 10 Gew.-%; |
| • n-Buten: | 0 Gew.-% bis 10 Gew.-%; |
| • Summe sonstige Stoffe: | 0 Gew.-% bis 5 Gew.-%. |

Es handelt sich somit im Wesentlichen um ein Gemisch aus C₃- und/oder C₄-Kohlenwasserstoffen.

Die enthaltenden Komponenten ergänzen sich zu 100 Gew.-%. Alle genannten Komponenten können enthalten sein, müssen es aber nicht. "Sonstige Stoffe" sind die zuvor nicht explizit aufgezählten Komponenten. Weiterhin gilt die Maßgabe, dass der Massenteil der Alkene in dem Einsatzstoffgemisch bezogen auf dessen Gesamtmasse 1 Gew.-% bis 10 Gew.-% beträgt, und dass das Einsatzstoffgemisch flüssig bei einem Druck zwischen 0.1^{∗}10⁵ Pa und 6.0^{∗}10⁵ Pa bereitgestellt wird.

Wichtig ist, dass in Gegenwart des ersten Katalysators die Dehydriertemperatur noch nicht erreicht wird. Deswegegen wird das mit dem Wasserstoff versetzte Einsatzstoffgemisch bzw. das daraus entstehende Intermediat erst allmählich an die Dehydriertemperatur herangeführt. Demnach wird das Verfahren durchgeführt in einem Apparat, der eine Aufheizzone und eine Reaktionszone aufweist, wobei der erste Katalysator in der Aufheizzone und der zweite Katalysator in der Reaktionszone angeordnet ist, und wobei das Einsatzstoffgemisch bzw. das Intermediat in der Aufheizzone so erhitzt wird, dass es mit einer Temperatur zwischen 450 °C und 760 °C in die Reaktionszone eintritt. Der Kontakt mit dem ersten festen Katalysator (also die Hydrierung) findet demnach in der Aufheizzone bei Temperaturen statt, bei denen die Dehydrierung nicht thermodynamisch bevorzugt ist. Typische Hydriertemperaturen liegen zwischen 20 °C und 220 °C.

Allerdings kann vorliegend die Hydriertemperatur höher liegen, dass das Einsatzstoffgemisch bei Reaktionsdruck der Dehydrierung verdampft werden muss. Die Hydriertemperatur kann also auch zwischen 220 °C und 450 °C liegen, etwa bei 350 °C. Entscheidend für die Abgrenzung zwischen Aufheizzone und Reaktionszone ist also das Erreichen eines thermodynamischen Zustands, bei der das Gleichgewicht zwischen Hydrierung und Dehydrierung kippt.

Ganz besonders bevorzugt wird das Verfahren isobarisch durchgeführt, das heißt, der Druck, der in der Dehydrierung herrscht, herrscht auch in der Hydrierung und in der Dosierung des Wasserstoffs und in der Verdampfung des Einsatzstoffgemisches, welches bereits bei Dehydrierdruck bereitgestellt wird. Druckverluste durch Strömung/Gasdynamik sind dabei zu vernachlässigen.

Ein geeigneter Apparat zur Durchführung des erfindungsgemäßen Verfahrens umfasst einen Zulauf für die Aufnahme eines flüssigen Einsatzstoffgemisches, welches unter einem Druck zwischen 0.1^{∗}10⁵ Pa und 6.0^{∗}10⁵ Pa steht, einen Verdampfer zum Verdampfen des Einsatzstoffgemisches durch Erhöhung dessen Temperatur, ein Organ zum Eindosieren von Wasserstoff in das verdampfte Einsatzstoffgemisch, eine Aufheizzone zum Aufheizen des verdampften Einsatzstoffgemisches bzw. eines daraus resultierenden Intermediats, Mittel zum Beheizen der Aufheizzone, eine Reaktionszone zum Kontaktieren des Intermediats mit einem zweiten festen Katalysator, in welcher der zweite feste Katalysator angeordnet ist und Mittel zum Beheizen der Reaktionszone auf eine Temperatur zwischen 450 °C und 760 °C. In der Aufheizstrecke des Apparats ist ein erster fester Katalysator anzuordnen und der gesamte Apparat ist für einen Druck zwischen 0.1^{∗}10⁵ Pa und 6.0^{∗}10⁵ Pa auszulegen, damit sich das Verfahren isobarisch ausführen lässt. Ein solcher Apparat ist ebenfalls Gegenstand der Erfindung.

Wie bereits oben geschildert, wird das Gleichgewicht zwischen Hydrierung und Dehydrierung mit höherer Temperatur in Richtung Dehydrierung geschoben. Wenn aber zusätzlich Wasserstoff eingespeist wird, verschiebt sich das Gleichgewicht wieder in Richtung Hydrierung. Das passiert auch bei hohen Temperaturen (zwischen 450 °C und 760 °C), bei denen sonst eigentlich eher die Dehydrierung betrieben wird. Deswegen ist es auch möglich, Hydrierung und Dehydrierung in einem Schritt an einem Katalysator bei Dehydrierbedingungen zu betrieben. Deswegen kann die Wasserstoffzugabe erfindungsgemäß auch in den Deyhdrierreaktor bei Dehydrierbedingungen erfolgen, wobei zu beachten ist, dass der Wasserstoff-Gehalt so limitiert ist, dass er in sehr engen Grenzen dem Olefingehalt im Feed entspricht (molares Verhältnis von Wasserstoff zu den im Einsatzstoffgemisch enthaltenen Alkenen liegt zwischen 0.8 : 1 und 1.2 : 1), damit nicht gegen das Gleichgewicht gearbeitet wird.

Das sich daraus ergebende einstufige Verfahren hat mit dem oben geschilderten zweistufigen Verfahren die kontrollierte Korrelation des Wasserstoffs in Bezug auf die Olefine gemein.

Ein entsprechendes Verfahren ist mithin ebenfalls ein Gegenstand der Erfindung. Es weist die folgenden Schritte auf:
a) Bereitstellen eines flüssigen Einsatzstoffgemisches bei einem Druck zwischen 0.1^{∗}10⁵ Pa und 6.0^{∗}10⁵ Pa, wobei das Einsatzstoffgemisch Alkane mit zwei bis fünf Kohlenstoffatomen sowie Alkene mit zwei bis fünf Kohlenstoffatome umfasst, und wobei der Massenteil der Alkene in dem Einsatzstoffgemisch bezogen auf dessen Gesamtmasse 1 Gew.-% bis 10 Gew.-% beträgt;
b) Verdampfen des Einsatzstoffgemisches durch Temperaturerhöhung;
c) Zugabe von Wasserstoff in das verdampfte Einsatzstoffgemisch dergestalt, dass das molare Verhältnis von Wasserstoff zu den im Einsatzstoffgemisch enthaltenen Alkenen zwischen 0.8 : 1 und 1.2 : 1 beträgt;
d) Kontaktierten des verdampften, wasserstoffhaltigen Einsatzstoffgemisches mit einem festen Katalysator bei einer Temperatur zwischen 450 °C und 760°C und einem Druck von 0.1^{∗}10⁵ Pa bis 6.0^{∗}10⁵ Pa unter Erhalt eines Produktgemisches, wobei der Massenteil der Alkene mit zwei bis fünf Kohlenstoffatomen in dem Produktgemisch bezogen auf dessen Gesamtmasse 30 Gew.-% bis 70 Gew.-% beträgt.

Als fester Katalysator wird dabei ein für die Dehydrierung geeignetes System eingesetzt, welcher sowohl die Hydrierung als auch die Dehydrierung katalytisch wirksam ist, vorzugsweise ein Katalysator, der ein Trägermaterial und darauf aufgebracht mindestens ein Element ausgewählt aus der Gruppe bestehend aus Nickel, Platin und Palladium umfasst. Das einstufige Verfahren lässt sich im Übrigen auch als zweistufiges Verfahren auffassen, bei dem in beiden Stufen der identische Katalysator eingesetzt wird. Dadurch wird der beiden Varianten zu Grunde liegende, gemeinsame Erfindungsgedanke deutlich.

Die Erfindung soll nun anhand eines vereinfachten Prozessfließbildes näher erläutert werden:

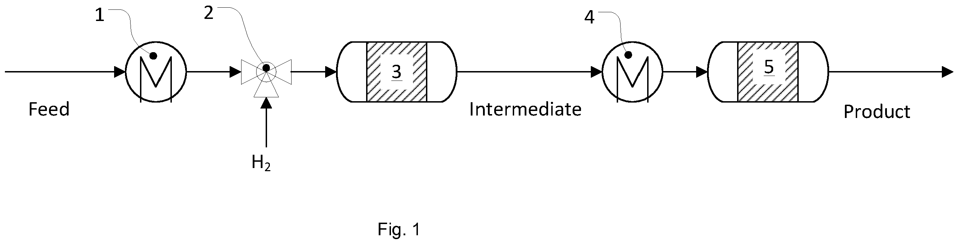

- Figur 1:: Prozessfließbild erfindungsgemäßes Verfahren.

Das Einsatzstoffgemisch *Feed* wird in flüssiger Form angeliefert und zwar auf dem Druckniveau der späteren Dehydrierung.

In einem Verdampfer 1 wird das Einsatzstoffgemisch verdampft. Dies geschieht durch Erwärmung. In einem Organ 2 wird dem verdampften Einsatzstoffgemisch Wasserstoff *H₂* zudosiert, und zwar möglichst genau die molare (stöchiometrische) Menge an im Einsatzstoffgemisch enthaltenen Alkene. Das gasförmige mit Wasserstoff angereicherte Einsatzstoffgemisch wird nun mit einem ersten festen Katalysator 3 in Kontakt gebracht. Dabei werden die im Einsatzstoffgemisch Feed enthaltenen Alkene mit dem zugeführten Wasserstoff *H₂* zu den korrespondieren Alkanen hydriert. Dabei wird ein Intermediat *Intermediate* erhalten, dessen Alken-Anteil nunmehr unter 1 Gew.-% liegt.

Spätestens jetzt wird das Intermediat in einem Wärmetauscher 4 auf ein für die Dehydrierung notwendiges Temperaturniveau (450 °C bis 760 °C) gebracht. Es ist aber auch möglich, schon bereits während der Hydrierung am ersten Katalysator 3 vorzuheizen, solange dadurch nicht die Gleichgewichtsreaktion in Richtung Dehydrierung verschoben wird. Es ist auch zu beachten, dass bereits durch die exotherme Hydrierung Reaktionswärme frei wird, welche in das Intermediat fließt.

Da die Temperatur des Einsatzstoffgemischs bzw. des daraus resultierenden Intermediats von der Zudosierung des Wasserstoffs im Organ 2 bis in den Wärmetauscher 4 zunimmt, ist dieser Bereich als eine Aufheizzone aufzufassen.

Das Intermediat wird nun durch Kontakt an einem zweiten Katalysator 5 einer Dehydrierung unterworfen, sodass ein Produktgemisch *Product* entsteht, welches wieder einen hohen Anteil an Alkenen (30 bis 70 Gew.-%) enthält. Dies geschieht in einer Reaktionszone des Verfahrens, die sich an die Aufheizzone anschließt. Die Grenze zwischen Aufheizzone und Reaktionszone liegt dort, wo die Temperatur so hoch ist, dass die Dehydrierung thermodynamisch gegenüber der Hydrierung bevorzugt ist. Da die Dehydrierung endotherm ist, sind entsprechend Mittel zum Beheizen der Reaktionszone notwendig; etwa ein Gasbrenner (nicht dargestellt).

Der erste Katalysator 3 und der zweite Katalysator 5 können unterschiedlich oder identisch sein. Aufheizzone und Reaktionszone können apparativ getrennt oder integriert sein. Bei einer isobarischen Fahrweise unterscheiden sie sich durch die Temperatur.

### Bezugszeichenliste

- 1:: Verdampfer
- 2:: Organ
- 3:: erster fester Katalysator
- 4:: Wärmetauscher
- 5:: zweiter fester Katalysator

- Feed:: Einsatzstoffgemisch
- Intermediate:: Intermediat
- Product:: Produktgemisch

## Patentansprüche

1. Verfahren zur Herstellung von Alkenen durch Dehydrierung von Alkanen, mit den folgenden Schritten:
a) Bereitstellen eines flüssigen Einsatzstoffgemisches bei einem Druck zwischen 0.1^{∗}10⁵ Pa und 6.0^{∗}10⁵ Pa, wobei das Einsatzstoffgemisch Alkane mit zwei bis fünf Kohlenstoffatomen sowie Alkene mit zwei bis fünf Kohlenstoffatomen umfasst, und wobei der Massenteil der Alkene in dem Einsatzstoffgemisch bezogen auf dessen Gesamtmasse 1 Gew.-% bis 10 Gew.-% beträgt;
b) Verdampfen des Einsatzstoffgemisches durch Temperaturerhöhung;
c) Zugabe von Wasserstoff in das verdampfte Einsatzstoffgemisch dergestalt, dass das molare Verhältnis von Wasserstoff zu den im Einsatzstoffgemisch enthaltenen Alkenen zwischen 0.8 : 1 und 1.2 : 1 beträgt;
d1) Kontaktierten des verdampften, wasserstoffhaltigen Einsatzstoffgemisches mit einem festen Katalysator bei einer Temperatur zwischen 450 °C und 760 °C und einem Druck von 0.1^{∗}10⁵ Pa bis 6.0^{∗}10⁵ Pa unter Erhalt eines Produktgemisches, wobei der Massenteil der Alkene mit zwei bis fünf Kohlenstoffatomen in dem Produktgemisch bezogen auf dessen Gesamtmasse 30 Gew.-% bis 70 Gew.-% beträgt;
oder
d2) Kontaktieren des verdampften, wasserstoffhaltigen Einsatzstoffgemisches mit einem ersten festen Katalysator und einem Druck zwischen 0.1^{∗}10⁵ Pa und 6^{∗}10⁵ Pa unter Erhalt eines Intermediats, wobei der Massenteil der Alkene in dem Intermediat bezogen auf dessen Gesamtmasse 0 Gew.-% bis 1 Gew.-% beträgt und wobei die Temperatur des verdampften, wasserstoffhaltigen Einsatzstoffgemisches und/oder des Intermediats erhöht wird; und
e) Kontaktierten des Intermediats mit einem zweiten festen Katalysator bei einer Temperatur zwischen 450 °C und 760 °C und einem Druck von 0.1^{∗}10⁵ Pa bis 6.0^{∗}10⁵ Pa unter Erhalt eines Produktgemisches, wobei der Massenteil der Alkene mit zwei bis fünf Kohlenstoffatomen in dem Produktgemisch bezogen auf dessen Gesamtmasse 30 Gew.-% bis 70 Gew.-% beträgt.

2. Verfahren nach Anspruch 1, **dadurch gekennzeichnet, dass** der erste Katalysator und der zweite Katalysator identisch sind.

3. Verfahren nach Anspruch 2, **dadurch gekennzeichnet, dass** der Katalysator ein Trägermaterial sowie mindestens ein Element ausgewählt aus den Gruppen 8, 9 und 10 des Periodensystems der Elemente gemäß IUPAC Konvention umfasst.

4. Verfahren nach Anspruch 2, **dadurch gekennzeichnet, dass** der Katalysator ein Trägermaterial sowie zumindest Zinn und/oder Zink umfasst.

5. Verfahren nach Anspruch 3 oder Anspruch 4, **dadurch gekennzeichnet dass** es sich bei dem Trägermaterial um Siliciumdioxid oder um Aluminiumoxid oder um eine Mischung aus Siliciumdioxid und Aluminiumoxid handelt.

6. Verfahren nach Anspruch 3 oder Anspruch 4, **dadurch gekennzeichnet dass** es sich bei dem Trägermaterial um ein Aluminat handelt, welches gebildet ist aus Aluminiumoxid und einem Erdalkalimetall.

7. Verfahren nach Anspruch 3 oder Anspruch 4, **dadurch gekennzeichnet dass** es sich bei dem Trägermaterial um Hydrotalcit handelt.

8. Verfahren nach Anspruch 1, **dadurch gekennzeichnet, dass** der erste Katalysator und der zweite Katalysator unterschiedlich sind.

9. Verfahren nach Anspruch 8, **dadurch gekennzeichnet, dass** der erste Katalysator ein Trägermaterial und darauf aufgebracht mindestens ein Element ausgewählt aus der Gruppe bestehend aus Nickel, Platin und Palladium umfasst.

10. Verfahren nach Anspruch 8, **dadurch gekennzeichnet dass** es sich bei dem Trägermaterial um Siliciumdioxid oder um Aluminiumoxid oder um eine Mischung aus Siliciumdioxid und Aluminiumoxid handelt.

11. Verfahren nach Anspruch 1 oder einem der Ansprüche 2 bis 10, **dadurch gekennzeichnet, dass** das Einsatzstoffgemisch die folgende, sich zu 100 Gew.-% ergänzende Zusammensetzung aufweist:
| | |
|---|---|
| • Propan: | 0 Gew.-% bis 50 Gew.-%; |
| • Isobutan: | 0 Gew.-% bis 100 Gew.-%; |
| • n-Butan: | 0 Gew.-% bis 100 Gew.-%; |
| • Propen: | 0 Gew.-% bis 10 Gew.-%; |
| • Isobuten: | 0 Gew.-% bis 10 Gew.-%; |
| • n-Buten: | 0 Gew.-% bis 10 Gew.-%; |
| • Summe sonstige Stoffe: | 0 Gew.-% bis 5 Gew.-%. |

12. Verfahren nach Anspruch 1 oder einem der Ansprüche 2 bis 11, durchgeführt in einem Apparat aufweisend eine Aufheizzone und eine Reaktionszone, wobei der erste Katalysator in der Aufheizzone und der zweite Katalysator in der Reaktionszone angeordnet ist, und wobei das Einsatzstoffgemisch bzw. das Intermediat in der Aufheizzone so erhitzt wird, dass es mit einer Temperatur zwischen 450 °C und 760 °C in die Reaktionszone eintritt.

13. Verfahren nach Anspruch 1 oder einem der Ansprüche 2 bis 12, **dadurch gekennzeichnet, dass** es isobarisch durchgeführt wird.

14. Apparat zur Durchführung eines Verfahrens nach Anspruch 12 oder 13, umfassend einen Zulauf für die Aufnahme eines flüssigen Einsatzstoffgemisches, welches unter einem Druck zwischen 0.1^{∗}10⁵ Pa und 6.0^{∗}10⁵ Pa steht, einen Verdampfer zum Verdampfen des Einsatzstoffgemisches durch Erhöhung dessen Temperatur, ein Organ zum Eindosieren von Wasserstoff in das verdampfte Einsatzstoffgemisch, eine Aufheizzone zum Aufheizen des verdampften Einsatzstoffgemisches bzw. eines daraus resultierenden Intermediats, Mittel zum Beheizen der Aufheizzone, eine Reaktionszone zum Kontaktieren des Intermediats mit einem zweiten festen Katalysator, in welcher der zweite feste Katalysator angeordnet ist und Mittel zum Beheizen der Reaktionszone auf eine Temperatur zwischen 450 °C und 760 °C, **dadurch gekennzeichnet, dass** in der Aufheizstrecke ein erster fester Katalysator angeordnet ist und dass der gesamte Apparat für einen Druck zwischen 0.1^{∗}10⁵ Pa und 6.0^{∗}10⁵ Pa ausgelegt ist.

## Claims

1. Process for preparing alkenes by dehydrogenation of alkanes having the following steps:
a) providing a liquid feedstock mixture at a pressure between 0.1^{∗}10⁵ Pa and 6.0^{∗}10⁵ Pa, wherein the feedstock mixture comprises alkanes having two to five carbon atoms and alkenes having two to five carbon atoms, and wherein the part by mass of alkenes in the feedstock mixture based on the total mass thereof is 1% by weight to 10% by weight;
b) evaporating the feedstock mixture by increasing the temperature;
c) adding hydrogen to the evaporated feedstock mixture such that the molar ratio of hydrogen to the alkenes present in the feedstock mixture is between 0.8 : 1 and 1.2: 1;
d1) contacting the evaporated, hydrogen-containing feedstock mixture with a solid catalyst at a temperature between 450°C and 760°C and a pressure of 0.1^{∗}10⁵ Pa to 6.0^{∗}10⁵ Pa to obtain a product mixture, wherein the part by mass of the alkenes having two to five carbon atoms in the product mixture based on the total mass thereof is 30% by weight to 70% by weight;
d2) contacting the evaporated, hydrogen-containing feedstock mixture with a first solid catalyst and a pressure between 0.1^{∗}10⁵ Pa and 6^{∗}10⁵ Pa to obtain an intermediate, wherein the part by mass of the alkenes in the intermediate based on the total mass thereof is 0% by weight to 1% by weight and wherein the temperature of the evaporated, hydrogen-containing feedstock mixture and/or of the intermediate is increased; and
e) contacting the intermediate with a second solid catalyst at a temperature between 450°C and 760°C and a pressure of 0.1^{∗}10⁵ Pa to 6.0^{∗}10⁵ Pa to obtain a product mixture, wherein the part by mass of the alkenes having two to five carbon atoms in the product mixture based on the total mass thereof is 30% by weight to 70% by weight.

2. Process according to Claim 1, **characterized in that** the first catalyst and the second catalyst are identical.

3. Process according to Claim 2, **characterized in that** the catalyst comprises a support material and at least one element from groups 8, 9 and 10 of the Periodic Table of the Elements according to IUPAC convention.

4. Process according to Claim 2, **characterized in that** the catalyst comprises a support material and at least tin and/or zinc.

5. Process according to Claim 3 or Claim 4, **characterized in that** the support material is silicon dioxide or aluminium oxide or a mixture of silicon dioxide and aluminium oxide.

6. Process according to Claim 3 or Claim 4, **characterized in that** the support material is an aluminate which has been formed from aluminium oxide and an alkaline earth metal.

7. Process according to Claim 3 or Claim 4, **characterized in that** the support material is hydrotalcite.

8. Process according to Claim 1, **characterized in that** the first catalyst and the second catalyst are different.

9. Process according to Claim 8, **characterized in that** the first catalyst comprises a support material and at least one element applied thereto selected from the group consisting of nickel, platinum and palladium.

10. Process according to Claim 8, **characterized in that** the support material is silicon dioxide or aluminium oxide or a mixture of silicon dioxide and aluminium oxide.

11. Process according to Claim 1 or any of Claims 2 to 10, **characterized in that** the feedstock mixture has the following composition adding up to 100% by weight:
| | |
|---|---|
| • Propane: | 0% by weight to 50% by weight; |
| • Isobutane: | 0% by weight to 100% by weight; |
| • n-Butane: | 0% by weight to 100% by weight; |
| • Propene: | 0% by weight to 10% by weight; |
| • Isobutene: | 0% by weight to 10% by weight; |
| • n-Butene: | 0% by weight to 10% by weight; |
| • sum of other substances: | 0% by weight to 5% by weight. |

12. Process according to Claim 1 or any of Claims 2 to 11, conducted in an apparatus having a heating zone and a reaction zone, wherein the first catalyst is arranged in the heating zone and the second catalyst is arranged in the reaction zone, and wherein the feedstock mixture or the intermediate is heated in the heating zone so that it enters the reaction zone at a temperature between 450°C and 760°C.

13. Process according to Claim 1 or any of Claims 2 to 12, **characterized in that** it is carried out isobarically.

14. Apparatus for carrying out a process according to Claim 12 or 13, comprising an intake for the feeding of a liquid feedstock mixture, which is under a pressure between 0.1^{∗}10⁵ Pa and 6.0^{∗}10⁵ Pa, an evaporator for evaporating the feedstock mixture by increasing the temperature thereof, a component for metering hydrogen into the evaporated feedstock mixture, a heating zone for heating the evaporated feedstock mixture or an intermediate resulting therefrom, means of heating the heating zone, a reaction zone for contacting the intermediate with a second solid catalyst in which the second solid catalyst is arranged and means of heating the reaction zone to a temperature between 450°C and 760°C, **characterized in that**, in the heating section, a first solid catalyst is arranged and that the entire apparatus is designed for a pressure between 0.1^{∗}10⁵ Pa and 6.0^{∗}10⁵ Pa.

## Revendications

1. Procédé pour la préparation d'alcènes par déshydrogénation d'alcanes, présentant les étapes suivantes :
a) préparation d'un mélange liquide de substances de départ à une pression entre 0,1^{∗}10⁵ Pa et 6,0^{∗}10⁵ Pa, le mélange de substances de départ comprenant des alcanes comprenant deux à cinq atomes de carbone ainsi que des alcènes comprenant deux à cinq atomes de carbone et la partie massique des alcènes dans le mélange de substances de départ, par rapport à sa masse totale, étant de 1% en poids à 10% en poids ;
b) évaporation du mélange de substances de départ par augmentation de la température ;
c) addition d'hydrogène dans le mélange de substances de départ évaporé de manière telle que le rapport molaire d'hydrogène aux alcènes contenus dans le mélange de substances de départ se situe entre 0,8:1 et 1,2:1 ;
d1) mise en contact du mélange de substances de départ évaporé, contenant de l'hydrogène, avec un catalyseur solide à une température entre 450°C et 760°C et à une pression de 0,1^{∗}10⁵ Pa à 6,0^{∗}10⁵ Pa avec obtention d'un mélange produit, la partie massique des alcènes comprenant deux à cinq atomes de carbone dans le mélange produit, par rapport à sa masse totale, étant de 30% en poids à 70% en poids ; ou
d2) mise en contact du mélange de substances de départ évaporé, contenant de l'hydrogène, avec un premier catalyseur solide et à une pression de 0,1^{∗}10⁵ Pa à 6^{∗}10⁵ Pa avec obtention d'un produit intermédiaire, la partie massique des alcènes dans le produit intermédiaire, par rapport à sa masse totale, étant de 0% en poids à 1% en poids et la température du mélange de substances de départ évaporé, contenant de l'hydrogène et/ou du produit intermédiaire étant augmentée ; et
e) mise en contact du produit intermédiaire avec un deuxième catalyseur solide à une température entre 450°C et 760°C et à une pression de 0,1^{∗}10⁵ Pa à 6,0^{∗}10⁵ Pa avec obtention d'un mélange produit, la partie massique des alcènes comprenant deux à cinq atomes de carbone dans le mélange produit, par rapport à sa masse totale, étant de 30% en poids à 70% en poids.

2. Procédé selon la revendication 1, **caractérisé en ce que** le premier catalyseur et le deuxième catalyseur sont identiques.

3. Procédé selon la revendication 2, **caractérisé en ce que** le catalyseur comprend un matériau support ainsi qu'au moins un élément choisi dans les groupes 8, 9 et 10 du système périodique des éléments selon la convention IUPAC.

4. Procédé selon la revendication 2, **caractérisé en ce que** le catalyseur comprend un matériau support ainsi qu'au moins de l'étain et/ou du zinc.

5. Procédé selon la revendication 3 ou la revendication 4, **caractérisé en ce qu'**il s'agit, pour le matériau support, de dioxyde de silicium ou d'oxyde d'aluminium ou d'un mélange de dioxyde de silicium et d'oxyde d'aluminium.

6. Procédé selon la revendication 3 ou la revendication 4, **caractérisé en ce qu'**il s'agit, pour le matériau support, d'un aluminate qui est formé à partir d'oxyde d'aluminium et d'un métal alcalino-terreux.

7. Procédé selon la revendication 3 ou la revendication 4, **caractérisé en ce qu'**il s'agit, pour le matériau support, d'hydrotalcite.

8. Procédé selon la revendication 1, **caractérisé en ce que** le premier catalyseur et le deuxième catalyseur sont différents.

9. Procédé selon la revendication 8, **caractérisé en ce que** le premier catalyseur comprend un matériau support et, appliqué sur celui-ci, au moins un élément choisi dans le groupe constitué par le nickel, le platine et le palladium.

10. Procédé selon la revendication 8, **caractérisé en ce qu'**il s'agit, pour le matériau support, de dioxyde de silicium ou d'oxyde d'aluminium ou d'un mélange de dioxyde de silicium et d'oxyde d'aluminium.

11. Procédé selon la revendication 1 ou selon l'une quelconque des revendications 2 à 10, **caractérisé en ce que** le mélange de substances de départ présente la composition suivante, se complétant à 100% en poids :
| | |
|---|---|
| propane : | 0% en poids à 50% en poids ; |
| isobutane : | 0% en poids à 100% en poids ; |
| n-butane : | 0% en poids à 100% en poids ; |
| propène : | 0% en poids à 10% en poids ; |
| isobutène : | 0% en poids à 10% en poids ; |
| n-butène : | 0% en poids à 10% en poids ; |
| somme des autres substances : | 0% en poids à 5% en poids. |

12. Procédé selon la revendication 1 ou selon l'une quelconque des revendications 2 à 11, réalisé dans un appareil présentant une zone de chauffage et une zone de réaction, le premier catalyseur étant disposé dans la zone de chauffage et le deuxième catalyseur étant disposé dans la zone de réaction et le mélange de substances de départ ou le produit intermédiaire étant chauffé dans la zone de chauffage de manière telle qu'il entre à une température entre 450°C et 760°C dans la zone de réaction.

13. Procédé selon la revendication 1 ou selon l'une quelconque des revendications 2 à 12, **caractérisé en ce qu'**il est réalisé de manière isobarique.

14. Appareil pour réaliser un procédé selon la revendication 12 ou 13, comprenant une alimentation pour recevoir un mélange liquide de substances de départ, qui se trouve sous une pression entre 0,1^{∗}10⁵ Pa et 6,0^{∗}10⁵ Pa, un évaporateur pour évaporer le mélange de substances de départ par augmentation de sa température, un organe pour le dosage d'hydrogène dans le mélange de substances de départ évaporé, une zone de chauffage pour chauffer le mélange de substances de départ évaporé ou un produit intermédiaire résultant de celui-ci, des moyens pour chauffer la zone de chauffage, une zone de réaction pour la mise en contact du produit intermédiaire avec un deuxième catalyseur solide, dans laquelle le deuxième catalyseur solide est disposé, et des moyens pour chauffer la zone de réaction à une température entre 450°C et 760°C, **caractérisé en ce qu'**un premier catalyseur solide est disposé dans la zone de chauffage et **en ce que** l'appareil dans sa totalité est conçu pour une pression entre 0,1^{∗}10⁵ Pa et 6,0^{∗}10⁵ Pa.
